# EUROPEAN PATENT SPECIFICATION

(11) **EP 2 582 247 B1**
(45) Date of publication and mention of the grant of the patent: **26.12.2018**
(21) Application number: 11725451.6
(22) Date of filing: 15.06.2011
(51) Int. Cl.: A01N 63/00, A01N 43/16, A01P 3/00

(54) **MICROBIAL PROCESS AND COMPOSITION FOR AGRICULTURAL USE**
MIKROBIELLES VERFAHREN UND ZUSAMMENSETZUNG FÜR DIE LANDWIRTSCHAFT
PROCÉDÉ MICROBIEN ET COMPOSITION À USAGE AGRICOLE

(30) Priority: 16.06.2010 US 355447 P
(43) Date of publication of application: 24.04.2013
(73) Proprietor: Agrinos AS, 1366 Lysaker (NO)
(72) Inventor: LÓPEZ-CERVANTES, Jaime, Cd. Obregón Sonora CP 85137 (MX); ROCHIN, Karl Reiner Fick, Navojoa, Sonora CP 85800 Mexico (MX)
(74) Representative: Sutcliffe, Nicholas Robert
(86) International application number: PCT/EP2011/059936
(87) International publication number: WO 2011/157747

(56) References cited:
- EP-A1- 0 328 383
- EP-A1- 1 142 988
- WO-A1-89/01288
- WO-A1-89/07395
- WO-A1-97/09879
- WO-A1-2009/031874
- WO-A1-2011/076759
- US-A- 4 915 944
- US-A1- 2004 228 844
- DATABASE WPI Week 199634 Thomson Scientific, London, GB; AN 1996-339040 XP002671744, & JP 8 157310 A (BANKAKU SONPO KK) 18 June 1996 (1996-06-18)
- DATABASE WPI Week 199732 Thomson Scientific, London, GB; AN 1997-347341 XP002671745, & JP 9 143013 A (YAESU SUISAN KAGAKU KOGYO KK) 3 June 1997 (1997-06-03)
- DATABASE WPI Week 200676 Thomson Scientific, London, GB; AN 2006-737669 XP002671746, & KR 2005 0117990 A (C & C SCI CO LTD) 15 December 2005 (2005-12-15)

## Description

### FIELD OF THE INVENTION

Microbial processes and microbial compositions are disclosed that enhance crop production, increase plant defensive processes, decrease the level of plant pathogens and reduce the amount of fertilizer used.

### BACKGROUND OF THE INVENTION

Microbes have previously been used in agriculture. Examples include those disclosed in US Patents 4,952,229; 6,232,270 and 5,266,096.

Chitin has also been used in agriculture either as a protein complex (US Patent 4,536,207) or in combination with various microbes (US Patents 6,524,998 and 6,060,429)

Chitosan in combination with other components has been used in agricultural applications. See e.g. US Patents 6,649,566; 4,812,159; 6,407,040; 5,374,627 and 5,733,851. It has also been used to treat cereal crop seeds. See US Patent 4,978,381. US Patent 6,524,998 also discloses that chitosan can be used in combination with specific microbes for agricultural use.

WO 97/09879 reports the use of chitosan formulations for treating the root systems of seedlings and young plants. WO 2009/031874 reports the agricultural use of a strain of Bacillus subtilis. EP 1142988 reports microorganisms of the genus Bacillus that contain chitin and/or chitosan in their cell walls and may be used for improving soil, for treating organic waste by fermentation, for fermenting soybeans, and for reducing bitterness. US 4915944 reports a biological control agent comprising mycoparasite Trichoderma harzianum T-315 (ATCC No. 20671) which has fungicidal activity against fungi of the genera Pythium, Rhizoctonia, Sclerotium and Fusarium.

Notwithstanding the foregoing, there is a need to provide improved microbial compositions and processes that improve crop yield and reduce the amount of conventional fungicides and insecticides used in agricultural and horticultural applications.

### SUMMARY OF THE INVENTION

Microbial compositions comprising at least two components are disclosed. The first component comprises HYTa which is a consortium of microbes derived from fertile soils and commercial sources. The second component comprises at least one of chitin, chitosan, glucosamine and amino acids. The various microbes in HYTa are capable of fixing nitrogen, digesting proteins and other biopolymers such as chitin and chitosan, providing protection against plant pathogens and supplementing the microbial flora of soil. The invention is defined in the appended claims.

Also disclosed are processes where the aforementioned microbial compositions or their components are used to treat soil, seeds, seedlings and/or plant foliage.

In preferred embodiments HYTa is activated in an aqueous solution for 24-168 hours to allow the microbes to grow and reproduce before being used in the process. The conditions of the incubation influence the overall initial properties of HYTa.

In a preferred embodiment, HYTa is activated in the presence of chitin. Chitin responsive microbes in HYTa proliferate in this environment. This results in HYTa that has all of the properties of HYTa. However, it has enhanced capability against chitin containing plant pathogens.

In a preferred embodiment, the HYTa is activated in the presence of chitin, chitosan, glucosamine and amino acids. In this embodiment, after growth, the HYTa may contain residual chitin, chitosan, glucosamine and/or amino acids. Under such circumstances, the culture constitutes the disclosed microbial composition and can be applied directly to soil, seed, seedlings or plant foliage. Alternatively, one or more second components can be added to supplement the second components already in the composition or to change the components present in the thus formed microbial composition.

In some disclosure herein the activated HYTa is combined with one or more second components and applied to the soil, seed, seedlings or plant foliage or the HYTa and the second component(s) are applied separately. Such second components include chitin, chitosan, glucosamine and amino acids.

The application of the disclosed microbial formulations allows for the elimination or significant reduction in the amount of fertilizer, fungicide and insecticide used in agricultural applications. In some disclosure herein the use of the microbial formulations results in a decrease in the amount of green house gas emissions.

Also disclosed is treated soil composition comprising soil treated with HYTa.

Also disclosed is treated plant comprising plant treated with HYTa.

Also disclosed are treated seeds, seedlings and plants comprising seed, seedling or plant treated with HYTa.

### BRIEF DESCRIPTION OF THE DRAWINGS

Figure 1 is a diagram of the test area involving the growth of durum wheat in Sonora, Mexico where HYTa and HYTb were used.
Figure 2 is the same diagram as Figure 3 and shows zones that were compromised and suffered impairment by external factors during the trial.
Figure 3 graphically depicts the results from treating the soil and foliage of durum wheat with HYTa and HYTb.
Figure 4 shows the yield of melons as a function of size for soil and foliage that was either not treated or treated with HYTa and HYTb.
Figure 5 shows the yield of potatoes having diameters greater than 42 mm that were treated with HYTa, HYTb and HYTc as compared to untreated potatoes.

### DETAILED DESCRIPTION

Microbial compositions comprising HYTa and a second component are disclosed. HYTa is a consortium of microbes derived from further soils and commercial sources. The second component comprises at least one of chitin, chitosan, glucosamine and amino acids. The various microbes in HYTa are capable of fixing nitrogen, digesting proteins and other biopolymers such as chitin and chitosan, providing protection against plant pathogens and supplementing the microbial flora of soil. The microbial compositions or their components are used to treat soil, seeds, seedlings and/or plant foliage.

### HYTa

As used herein, the term "HYTa" refers to a consortium of microbes derived from fertile soil samples and commercial sources. HYTa was deposited with the American Tissue Type Culture (ATTC), Rockville, Maryland, on May 19, 2010 with an assigned deposit designation of PTA-10973.

Table 1 identifies some of the microbes in HYTa that are believed to be responsible for the beneficial effects observed when it is used to treat soil and/or foliage.

**Table 1**

| **Bacteria** |
|---|
| **I. Azotobacter** |
| *1. Azotobacter vinlandii* |

| **II. Clostridium** |
|---|
| *1. Clostridium pasteurianum* |
| *2. Clostridium beijerinckii* |
| *3. Clostridium sphenoides* |
| *4. Clostridium bifermentans* |

| **III. Lactobacillus** |
|---|
| *1. Lactobacillus paracasei ss. paracasei* |
| *2. Lactobacillus acidophillus* |
| *3. Lactobacillus delbrueckii ss. Bulgaricus* |
| *4. Lactobacillus brevis* |

| **IV. Bacillus** |
|---|
| *1. Bacillus amyloliquefaciens (Bacillus subtilis* ( (SILoSil® BS)*)* |
| *2. Bacillus thuringiensis var. kurstakii (Bacillus thuringiensis* (Strains HD-1)) |
| *3. Bacillus thuringiensis var. canadensis (Bacillus cereus* group) |
| *4. Bacillus pasteurii (Bacillus cereus* group) |
| *5. Bacillus sphaericus* (subgroup I, III, and IV) |
| *6. Bacillus megaterium* (subgroup A) |

| **V. Acetobacter or Gluconacetobacter** |
|---|
| *1. Acetobacter aceti ss. liquefaciens* |
| *2. Acetobacter aceti ss. xylimum* |

| **VI. Enterococcus** |
|---|
| *1. Enterococcus faecium (subgroup A)* |

| **VII. Pediococcus** |
|---|
| 1. *Pediococcus pentosaceus* |

| **VII. Rhizobium** |
|---|
| 1. *Rhizobium japonicum* |

| **Fungi** |
|---|
| **I. Saccharomyces** |
| 1. *Saccharomyces cerevisiae* |

| **II. Penicillium** |
|---|
| 1. *Penicillium roqueforti* |

| **III. Monascus** |
|---|
| 1. *Monascus ruber* |

| **IV. Aspergillus** |
|---|
| 1. *Aspergillus oryzae* |

| **V. Trichoderma** |
|---|
| 1. *Trichoderma harzianum* (TRICHOSIL) |

| **Plantae** |
|---|
| **I. Arthrospiro** |
| 1. *Arthrospira platensis* |

| **II. Ascophyllum** |
|---|
| 1. *Ascophyllum nodosum* |

Other microorganisms contained in HYTa: *Nitrobacter, Nitrosomonads, Nitrococcus, Pseudomonas, Micrococcus luteus, Actinomycete, Azotobacter vinelandii, Lactobacillus casei, Trichoderma harzianum, Bacillus licheniformis, Pseudomonas fluorescens* and *Streptomyces.*

Active microbes in HYTa include nitrogen-fixing microorganisms native to soil. These are *Azotobacter vinelandii* and *Clostridium pasteurianum. Bacillus subtilis* provides enzymes for breaking down plant residue. *Bacillus cereus* provides additional enzymes to break down plant residue and penicillinase to decease unwanted bacteria. *Bacillus megaterium* degrades complex sugars after crop residue breakdown. *Lactobacillus* provides food for the microbes in HYTa and controls the pH of the environment. The *Nitrobacter* organisms oxidize ammonia to nitrite (NO₂) while the *Nitrosomonas* microbes oxidize nitrite to nitrate (NO₃).

An important property of HYTa is the fixation of atmospheric nitrogen. The nitrogen fixing capability of the microbes in HYTa is enhanced by the assistance of other organisms in HYTa. Nitrogen fixation requires that phosphorous (P), potassium (K) and carbon (C) be available. HYTa contains microbes that are able to decompose P, K, and C within the soil. In addition, the nitrogen fixing bacteria provide a source of nitrogen for the other microbes in HYTa.

Nitrogen fixation may occur in a non-symbiotic manner by the bacteria *Nitrosomonas, Nitrobacter, Azotobacter vinelandii, and Clostridium pasteurinum* present in HYTa or in a symbiotic manner as occurs in root nodules by way of the *Rhyzobium* bacteria.

The carbon required by the nitrogen fixing microbes in HYTa is provided by the C decomposers which convert the complex organic compounds in soil into simple compounds such as sugars, alcohols, and organic acids. The C decomposers include many of the above identified microbes.

Phosphorus is necessary for the nitrogen fixing microbes to proliferate and is obtained from the metabolic activity of the P decomposers which convert immobilized phosphorus in the soil into a bio-available phosphorus nutrient. P decomposers in HYTa include *Azotobacter, Bacillus subtilis, Pseudomonas fluorescens* and *Micrococcus luteus.*

The potassium required by the nitrogen fixers is provided by the K decomposer microbes present in HYTa which activate the potassium from the soil. K decomposers in HYTa include *Pseudomonas fluorescens.*

Three important microbes in HYTa are *Bacillus subtilis* (SILoSil® BS) *Bacillus thuringiensis* strains HD-1 and HD-73 (SILoSil® BT), and *Trichoderma harzianum* (TRICHOSIL). These organisms are present ATTC deposit PTA-10973. They were originally obtained from Biotecnologia Agroindustrial S.A. DE C.V., Morelia, Michoacan, Mexico.

*Bacillus subtilis* ((SILoSil® BS) is a Gram positive bacterium which is mesophilic and grows at an optimum temperature between 25 and 35°C. It is aerobic and can grow in anaerobic conditions and utilizes a wide variety of carbon sources. It contains two nitrate reductases, one of which is utilized for nitrogen assimilation. It is capable of secreting amylase, proteases, pullulanases, chitinases, xilanases and lipases.

*Bacillus thuringiensis* (Strains HD-1 and HD-73 (SILoSil® BT)) are Gram Positive anaerobic facultative bacteria, in the form of a peritrichous flagella. Strains HD-1 and HD-73 synthetizes crystals with diverse geometric forms of proteic and insecticide activity during the spore period. Strains HD-1 and HD-73 secret exochitanases when in a chitin containing medium and can be utilized for the degradation of the crustacean residues during the production of chitooligosaccharides.

*Trichoderma harzianum* (TRICHOSIL) is a saprophyte fungus. It exhibits antibiotic action and biological competition and for this reason has biological control properties. It produces enzymes that degrade cell walls or a combination of such activities. It produces glucanases, chitinases, lipases, and extracellular proteases when it interacts with some pathogenic fungi, such as *Fusarium.*

As shown above the metabolism of each group of bacteria are closely interdependent and live in a close symbiotic association for the proper performance of HYTa.

Besides carbon, hydrogen, phosphorus, potassium, sulfur and various trace elements, a mix of special growth factors, such as B complex, free L-amino acids, and ultra soluble trace elements are important for optimal bacterial growth. Fermenting yeasts are incorporated into HYTa to provide these components. The N₂ fixing process requires large amounts of ATP. The amount of ATP naturally present is not enough to fuel biological N₂ fixation. The fermentation of the yeast in HYTa compensates for the large energy deficit. During fermentation, organic acids are formed in the respiratory process and together with the phosphorous released by the P decomposers, form ATP. The ATP is used in the biological nitrogen fixation process.

HYTa contains enzymes and beneficial soil microorganisms that replace those that have been depleted due to the excessive use of chemicals which results in diminishing crop yields. By increasing the microbial activity in the soil with HYTa, the bacteria causes the nutrients and micro-elements to be absorbed (mineralized) more efficiently and effectively by plants.

Humus is transformed by some of the microorganisms in HYTa that impregnate both the soil and the radical apparatus of the plant. This process provides increased nutrition to the plant. This increases the nutrients and the essential elements available in the soil that can be absorbed by plants.

The use of HYTa alone or in combination with chitin, chitosan, glucosamine and/or amino acids (1) provides nutrients and elements in the soil that increase crop yields by 25-55%, (2) reduces green house gas emissions, (3) increases the efficiency of mineral fertilizers (3) reduces the use of conventional fungicides and other pesticides, (4) increases the production of plant growth regulators, (5) improves soil structure, tilth, and water penetration and retention, (6) cleans up chemical residues and (7) shifts soil pH toward neutral pH.

### MICROBIAL COMPOSITIONS

HYTa can be used, alone or in combination, with one or more components selected from the group of one or more amino acids, chitin, chitosan and/or glucosamine. In some cases, Acetyl-D-glucosamine can be included in the microbial composition. The microbial composition includes any and all combinations of the aforementioned components. Particularly preferred combinations include: (1) HYTa and chitin; (2) HYTa and chitosan; (3) HYTa and glucosamine; (4) HYTa and amino acids; (5) HYTa, chitin and amino acids; (6) HYTa, chitin, chitosan and amino acids; (7) HYTa, chitosan, glucosamine and amino acids; (8) HYTa, chitosan and glucosamine and (9) HYTa, chitin, chitosan, glucosamine and amino acids, the latter being particularly preferred.

When HYTa is grown in the presence of chitin, chitosan and/or amino acids it may contain residual chitin, chitosan and/or amino acids. Under such circumstances, the HYTa culture constitutes the disclosed microbial composition and can be applied directly to soil, seed, seedlings or plant foliage. Alternatively, one or more of the second components can be added to supplement the second components in the composition or to change its composition.

As used herein, the term "amino acids" refers to a composition containing two or more amino acids. Amino acids include tryptophan, histidine, threonine, tyrosine, valine, methionine, isoleucine, leucine, phenylalanine, lysine, aspartic acid, cysteine, glutamic acid, glutamine, serine, glycine, alanine, proline, asparagine and arginine. In preferred embodiments, amino acids are provided by use of HYTb (See below).

As used herein, the term "chitin" refers to a biopolymer consisting predominantly of repeating units of beta-1-4-linked N-acetyl-D-glucosamine. Chitin is found in the natural environment as a primary structural material of the exoskeleton of animals such as *Arthropoda,* e.g., crustaceans, insects, spiders, etc., *Mollusca,* e.g., snails, squid, etc., *Coelentara,* e.g., organisms such as hydoids and jellyfish, and *Nematoda,* such as unsegmented worms. Chitin is also found in various fungi including members of the genus *Fusarium.* Chitin can be extracted from these natural sources by treatment with alkali, or by a biodegradation process. The molecular weight of chitin varies depending on its source and method of isolation. In preferred embodiments, the chitin is derived as a solid from the biodegradation of chitin containing *Arthropods* as described in the Bioderpac applications. It is preferred that the chitin have a diameter of about 50 to 75 microns to facilitate its application via drip and spray irrigation systems.

As used herein, the term "chitosan" is a polysaccharide consisting predominantly of repeating units of D-glucosamine. Chitosan is obtained by deacetylation of chitin. The degree of deacetylation as compared to chitin is preferably greater than 50%, 60%, 70%, 80%, 85%, 90% and 95%. It is preferred that the level of deacetylation be sufficient to render the chitosan water soluble at acidic pH. The molecular weight of chitosan varies depending on its source and method of isolation. Chitosan includes chitosan oligomers. In preferred embodiments, chitosan is precipitated at pH 9.0 from the aqueous fraction obtained from the biodegradation of chitin containing *Arthropods* such as described in the Bioderpac applications.

As used herein, the term "chitosan oligomer" refers to chitosan having 2 or more repeating units of D-glucosamine and, in the case of incomplete deacetylation of chitin, one or more units of N-acetyl-D-glucosamine. In preferred embodiments, the chitosan oligomers are derived from the aqueous fraction generated in the biodegradation of chitin containing *Arthropods* such as described in the Bioderpac applications. In some embodiments chitosan oligomers are used as the second component of the microbial composition.

As used herein, the term "glucosamine" refers to an amino monosaccharide. In preferred embodiments it is the sugar residue that forms the backbone of the biopolymers chitin and chitosan. Glucosamine is present in the aqueous fraction generated during the biodegradation of chitin containing *Arthropods* such as described in the Bioderpac applications. Glucosamine induces plants to make chitinase as a defense to chitin containing pathogens.

### HYTb and HYTc

As used herein, the term "HYTb" refers to the aqueous fraction and "HYTc" refers to the solid fraction obtained from the biodegradation of Arthropods such as shrimp waste. derived from the biodegradation or chitin containing *Arthropods* such as described in US Patent Application Serial No. 61/289,706, filed 12/23/09 entitled "Biodegradation of Crustacean By-products", US Patent Application Serial No. 61/299,869, filed 1/29/10 entitled "Biodegradation Process and Microbial Composition" and US Patent Application Serial No. 61/355,365 filed June 16, 2010 entitled "Biodegradation Process and Composition" .

Briefly, in the arthropod biodegradation process a microbial composition is used to degrade the arthropod or waste components of the arthropod. It is a lactic acid fermentation process. The microbial composition contains microbes that produce enzymes that can degrade the chitin containing components of the arthropod to chitin, chitosan, N-acetyl glucosamine and glucosamine. It also contains microbes that produce enzymes that can degrade proteins and fats to produce amino acids and lipids. A preferred microbial composition for arthropod degradation is referred to as HQE. HQE was deposited with the American Type Culture Collection (ATCC) Manassas, VA, USA on April 27, 2010 and given Patent Deposit Designation PTA-10861.

In a preferred embodiment, the marine arthropod is a crustacean and the preferred crustacean is shrimp. Shrimp by-product comprises shrimp cephalothorax and/or exoskeleton.

In the biodegradation process, it is preferred that the fermentation be facultative aerobic fermentation. It is also preferred that the fermentation is carried out at a temperature of about 30°C to 40°C. The pH is preferably less than about 6, more preferably less than about 5.5. However, the pH should be maintained above about 4.3. The fermentation is carried out for about 24-96 hours. In some embodiments, the fermentation is carried out for about 24-48 hours and more preferably 24-36 hours. These fermentation times are far shorter than the typical prior art fermentation times of 10 to 15 days to achieve substantially the same amount of digestion, albeit without detectable formation of chitosan and glucosamine.

The separation of the mixture is preferably by centrifugation. (e.g. about 920 g). Gravity separation can also be used but is not preferred because of the time required to achieve separation.

The mixture separates in to three fractions: solid, aqueous and lipid. The solid fraction comprises chitin and is designated HYTc. The aqueous fraction comprises protein hydroysate, amino acids, chitosan and glucosamine and is designated HYTb. The lipid fraction comprises sterols, vitamin A and E and carotenoid pigments such as astaxanthine.

It is preferred that HQE be used in the biodegradation process. In other embodiments, it is preferred that previously prepared HYTb be added to HQE or the fermentation broth. As described above, HYTb contains amino acids, chitosan, glucosamine and trace elements including calcium, magnesium, zinc, copper, iron and manganese. HYTb also contains enzymes such as lactic enzymes, proteases, lipases, chitinases, lactic acid, polypeptides and other carbohydrates. HYTb can also contain dormant microorganisms from a prior biodegradation process. Such microorganisms can become reactivated and, in combination with HQE, contribute to a more robust biodegradation process as compared to when HQE is used by itself as otherwise described herein

More particularly, the process includes the following steps:
a. Activation of the microbial cells in a sugar base solution to enhance its growth and the biomass formation.
b. Milling of the shrimp by-products (cephalthorax and exosqueleton) to make a homogeneous paste.
c. Homogeneous mixing of the shrimp by-product paste with at least 10% of the activated inoculum.
d. Adjustment of the pH values to less than 6.0 in the mixture using a citric acid solution to inhibit the growth of micro organisms and to promote the development of microbial cells that constitute the inoculum.
e. Fermentation of the mixture in a non continuous agitated system at temperatures within a range of 30 to 40°C at least for at least 96 hours maintaining pH at less than 5.0. The pH is monitored periodically. If the pH rises above 5.0, a citric acid buffer is added in an amount to maintain the pH below 5.0.
f. Centrifugation of the ferment to separate the three principal fractions: chitin, liquid hydrolysate and pigmented paste.
g. Rinsing of the crude chitin and recollection of the rinse water to recuperate fine solids or minerals.
h. Drying of the chitin and storage.
i. Drying and storage of the liquid hydrolysate.
j. The pigmented paste (lipid fraction) is stored in closed recipients for conservation.

The process and operational fundamentals are better understood with reference to the following detailed description.

### Activation of microbial cells

A microbial composition as disclosed herein is used as inoculum. The inoculum of HQE has a concentration of microbes of about 2.5 to 3.0% (w/v). HQE is activated by dilution to 5% in sugar cane solution (3.75% final concentration of sugar cane), and incubated at 37 °C for 5 days. HYTb (10 ml per liter of culture) is preferably added to provide a source of minerals and naturally derived amino acids. The cellular growth of the microorganisms was estimated by optical density measured at 540 nm. The activation is complete at an optical density of about 1.7. The concentration of microbes after activation is about 1.9 to 3.0% (w/v).

### Preparation of samples

The shrimp by-products samples are obtained from shrimp processing plants. Slightly thawed and minced residue (1500 g by batch) is mixed with 99 grams of sugar cane (final concentration 6.6% wt%) and 85.5 ml of activated HQE 5% (v/w) (optical density of cell = 1.7). Then the pH is adjusted to 5.5 using 2 M citric acid.

### Fermentation control

The mixture is incubated at 36 °C with a non continuous agitation for 96 h. During the fermentation process, the pH is monitored by using a potentiometer, and the total titratable acidity (TTA, %) was determined by titration with 0.1 N NaOH until a pH of 8.5 is obtained. The TTA is expressed as a percentage of lactic acid.

### Conditions of separation

The fermentation product is a viscous silage which has an intense orange color, due to the astaxanthine presence. The ensilage is centrifuged (5 °C) at 1250 rpm (930g) for 15 min to obtain the chitin, the liquid hydrolysates, and the pigment paste. The upper phase (pigment paste) is separated manually. The liquid hydrolysates are separated by decantation, and the sediment that constitutes the raw chitin is washed with distilled water to separate fine solids. The resulting liquid is collected and dried. The raw chitin, liquid hydrolysates and fine solids are dried at 60 °C. All the fractions are stored to protect them from light.

Other microbial compositions for the production of HYTb and HYTc are set forth in the following Table 2.

**Table 2**

| **Culture Composition** | | | | | | | | | | |
|---|---|---|---|---|---|---|---|---|---|---|
| **Microorganism** | **1** | **2** | **3** | **4** | **5** | **6** | **7** | **8** | **9** | **10** |
| *Bacillus subtilis* | X | X | X | X | | X | X | X | | X |
| *Bacillus cereus* | X | X | | X | | X | | X | | X |
| *Bacillus megaterium* | X | X | | | | | | | | |
| *Azotobacter vinelandii* | X | X | | X | | X | | X | | X |
| *Lactobacillus acidophilus* | X | X | X | X | X | | X | X | X | |
| *Lactobacillus casei* | X | X | | X | X | | | X | X | |
| *Trichoderma harzianum* | X | X | X | X | | X | X | X | | X |
| *Rhizobium japonicum* | X | X | | X | | X | | X | | X |
| *Clostridium pasteurianum* | X | X | | | X | X | | | X | X |
| *Bacillus licheniformis* | X | X | X | | X | X | X | | X | X |
| *Pseudomonas fluorescens* | X | X | X | | X | X | | | | |
| *Bacillus thuringiensis* | X | | | | | X | X | X | X | X |
| *Streptomyces* | X | | | | X | X | X | X | X | X |
| *Nitrobacter* | X | | | | | | X | X | X | X |
| *Micrococcus* | X | | | | | | X | X | X | X |
| *Proteus vulgaris* | X | | | | | | X | X | X | X |

These microorganisms are preferably derived from HQE and are referred to as *Bacillus subtilis* ((SILoSil® BS), *Bacillus cereus* (Bioderpac, 2008), *Bacillus megaterium* (Bioderpac, 2008), *Azotobacter vinelandii* (Bioderpac, 2008), *Lactobacillus acidophilus* (Bioderpac, 2008), *Lactobacillus casei* (Bioderpac, 2008), *Trichoderma harzianum* (TRICHOSIL), *Rhizobium japonicum* (Bioderpac, 2008), *Clostridium pasteurianum* (Bioderpac, 2008), *Bacillus licheniformis* (Bioderpac, 2008), *Pseudomonas fluorescens* (Bioderpac, 2008), *Bacillus thuringiensis* strains HD-1 and HD-73 (SILoSil® BT), *Streptomyces* (Bioderpac, 2008), *Micrococcus* (Bioderpac, 2008), *Nitrobacter* (Bioderpac, 2008) and *Proteus* (Bioderpac, 2008). Each of these organisms can be readily isolated from HQE and recombined to form the disclosed microbial composition to degrade arthropods to make HYTb and HYTc.

HYTb contains amino acids (about 12 wt%), chitosan (about 1.2 wt%), glucosamine (about 1 wt%) and trace elements (about 6 wt%) including calcium, magnesium, zinc, copper, iron and manganese. It also contains enzymes such as lactic enzymes, proteases, lipases, chitinases among others, lactic acid, polypeptides and other carbohydrates. The specific gravity of HYTb is typically about 1.050-1.054. The average amino acid content in HYTb for certain amino acids is set forth in Table 2.

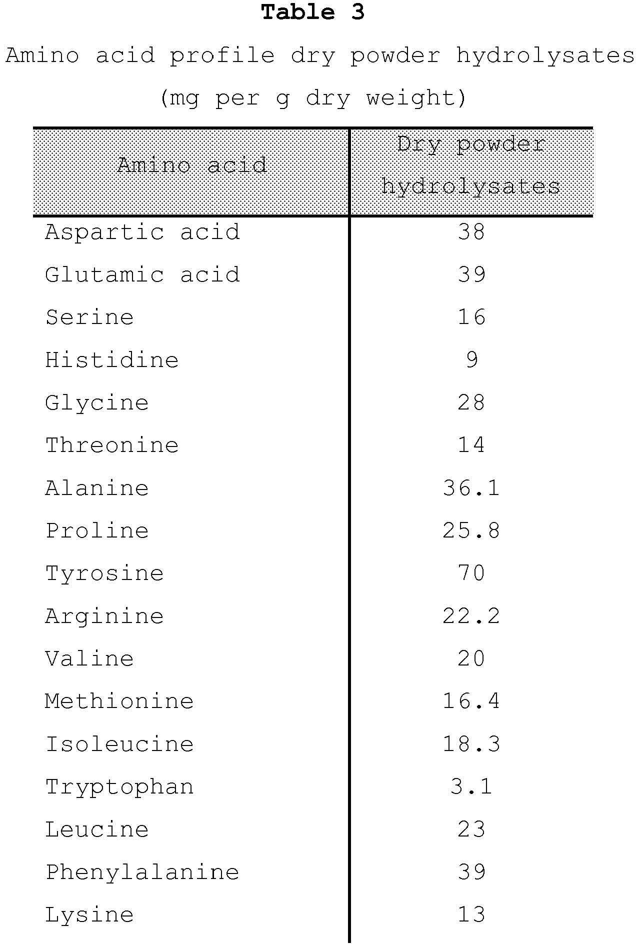

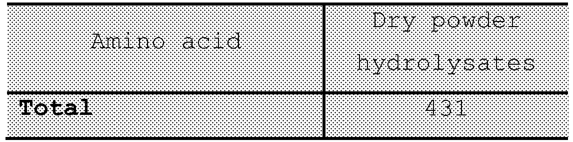

In some embodiments, HYTb can constitute a second component that is either combined with HYTa or used separately as a soil amendment and/or as a foliage spray.

The primary component of HYTc is chitin. It has an average molecular weight of about 2300 daltons and constitutes about 64 wt% of the composition. About 6 % of HYTc contains minerals including calcium, magnesium, zinc, copper, iron and manganese, about 24 wt% protein and 6% water. It has a specific gravity of about 272 Kg/m³. In some embodiments, HYTc can constitute a second component that is either combined with HYTa or used separately as a soil amendment and/or as a foliage spray.

HYTa is preferably used with HYTb and HYTc either in combination or separately as a soil amendment or foliage spray.

The microbes in HYTa require the trace elements calcium, magnesium, sulfur, boron, manganese, zinc, molybdenum, iron, copper, sodium, and silicon. These important trace elements can be often obtained from toxic chemical reactions which are not suitable for organic certified products. Accordingly, it is preferred that these trace elements be obtained from an organic source such as HYTb and/or HYTc.

### Activation of HYTa

The aforementioned microbial compositions can be used to treat soil, seeds, seedlings and/or plant foliage. However, HYTa is first activated before use.

In preferred embodiments, HYTa is activated by incubating an inoculum of HYTa in an aqueous solution for 24-168 hours to allow the microbes to grow and reproduce before being used in the process of treating soil, seeds, seedlings and/or plant foliage. The conditions of the incubation influence the overall initial properties of HYTa.

In one embodiment, an inoculum of HYTa is diluted with water in a ratio of 1/100 and allowed to incubate at a temperature of approximately 36°C at a pH of 6.8-7.1 for about 24 to about 168 hours (7 days). HYTb can optionally be used during this activation. The nitrogen fixing microbes *Azotobacter vinelandii* and *Clostridium pasteurianum* proliferate under reduced nitrogen growth conditions. In addition, as the oxygen concentration decreases, *Lactobacilli,* including *Lactobacillus acidophilus* and *Lactobacillus casei,* proliferate. The colony forming units (CFUs) for some of the bacteria in activated HYTa are set forth in Table 3:

**Table 4**

| | |
|---|---|
| *Azotobactervinelandii* | 101,050,000 Cfu/mL |
| *Clostridium pasteurianum* | 104,275,000 Cfu/mL |
| *Bacillus subtilis* | 1,100,000 Cfu/mL |
| *Bacillus cereus* | 25,000 Cfu/mL |
| *Bacillus megaterium* | 10,000 Cfu/mL |
| *Lactobacillus* | 500,000 Cfu/mL |
| *Nitrobacter* | 5,000 Cfu/mL |
| *Nitrosomonas* | 2,500 Cfu/mL |
| **Total** | **206,967,000Cfu/mL** |

The HYTa obtained after this incubation retains the beneficial properties of HYTa but is particularly suited as a soil amendment for treatment of nitrogen-depleted soils given the nitrogen-fixation capabilities of *Azotobacter vinelandii* and *Clostridium pasteurianum.*

If soil pathogens such as filamentous fungi from the genus *Fusarium* or nematodes are present, or believed to be present, HYTa can be activated under substantially the same conditions but in the presence of chitin. The chitin stimulates the expansion of the chitin responsive microbes such as *Pseudomonas fluorescens, Trichoderma harzianum, Bacillus thuringiensis, Streptomyces sp., Nitrobacter sp., Micrococcus sp.,* and *Bacillus subtilis.* HYTa obtained under these conditions has an antifungal, fungicidal, antinematode, nematodicidal and insecticidal properties to the extent such pathogens contain chitin. Such microbial compositions can be applied directly to the soil or to seed, seedlings and/or plant foliage. Such microbial compositions also have the ability to fix nitrogen as in the aforementioned incubation in the absence of chitin.

In addition to incubating with chitin, HYTa can be activated with chitin and amino acids. A preferred source of chitin is HYTc. When HYTc is used the protein and minerals in HYTc are also present during the activation.

Further, HYTa can be activated in the presence of amino acids and chitosan. A preferred source of amino acids and chitosan is HYTb. When HYTb is used glucosaime and the other components of HYTb are also present during the activation.

Optionally, HYTa can be incubated with chitin, amino acids and chitosan. A preferred source of chitin is HYTc. A preferred source for amino acids and chitosan is HYTb. When HYTb and HYTc are used the other components in these formulations are also present during activation.

### Use of Activated HYTa

Activated HYTa can be used alone or in combination with other components such as chitin, chitosan, glucosamine and amino acids to treat soil, seed, seedlings or foliage. In some embodiments, combinations of these components can be applied as a mixture. In other embodiments, they can be applied separately. In still other embodiments, the components can be applied at different times.

In one embodiment, activated HYTa can be applied to soil, seeds or seedlings, or used in foliar applications by direct application to foliage. However, when plant pathogens are present, it is preferred that microbial composition comprises activated HYTa, chitin and/or chitosan. Alternatively, the HYTa can be activated in the presence of chitin. Chitosan is known to have bactericidal, fungicidal, and antiviral properties, as well as its ability to stimulate plant growth and to induce plant resistance to pathogens. In other embodiments, glucosamine is a part of the microbial composition

In a preferred embodiment, the activated HYTa alone or in combination with chitin (preferably HYTc) and/ or chitin, chitosan, and amino acids (preferably HYTb and HYTc), is applied to soil, seeds, seedlings and/or foliage. It is preferred that HYTa be used in combination with chitin, chitosan, glucosamine and amino acids. HYTc is the preferred source of chitin while HYTb is the preferred source of chitosan, glucosamine and amino acids However, the components of the microbial composition namely HYTa, chitin, chitosan, glucosamine and amino acids can be applied separately or in any combination or sub-combination. They can be applied at the same time or sequentially, in any given order. However, the preferred mode of application is to initially apply all at the same time. The application of the foregoing components provide for the direct treatment of plant pathogens, the induction of plant pathogen resistance pathways, and the nourishment of the HYTa microbes, the indigenous nonpathogenic soil flora, and the plant.

When soil is initially treated with a microbial composition comprising activated HYTa alone, the microbes present in the composition have an opportunity to populate the soil and to alter its taxonomic composition. In some situations, the initial colonization by HYTa provides little or no nutrients to the plant. In such instances, it is important to maintain a nutrient reserve to sustain both the growth of the microbes while colonizing the rizosphere and the growth of the plants in the soil. It may be necessary to repeat the application of HYTa, depending on the plant's growth cycle and nutritional regime. In other cases, it may be sufficient to provide additional applications of amino acids, chitin and/or chitosan, eg. HYTb and HYTc, to the previously treated soil.

When HYTa is used in combination with, for example, HYTb and HYTc, addition nutrients are available to the HYTa microbes and the plants present in the treated soil.

Table 5 sets forth a typical fourteen week program for the application of HYTa, HYTb and HYTc to drip irrigated crops cultivated in soil. The values are per hectare. For HYTa and HYTb, the values represent liters per week. For HYTc, the values represent kilograms per week.

**Table 5**

| **Lts/kg/ Week** | **W1** | **W2** | **W3** | **W4** | **W5** | **W6** | **W7** | **W3** | **W9** | **W10** | **W11** | **W12** | **W13** | **W14** |
|---|---|---|---|---|---|---|---|---|---|---|---|---|---|---|
| **HYT-A** | 3 | 0 | 0 | 1 | 0 | 1 | 0 | 1 | 0 | 1 | 0 | 1 | 0 | 1 |
| **HYT-B** | 10 | 5 | 0 | 3 | 2 | 3 | 2 | 3 | 2 | 3 | 2 | 3 | 2 | 3 |
| **HYT-C** | 1 | | | 1 | | | | 1 | | | | 1 | | |

The pulse in which the microbial composition is injected to the irrigation system should be one in which the microbial composition is able to reach the root system and stay there over night while the system is off. For maximum performance of HYTc, it should be applied at the same time as a mixture with HYTa. The protocol should be continued as long as the plant continues in production. This protocol covers all plant stages including germination, root formation, plant growth, flowering, fruit setting, fruit formation harvesting and re-harvest. This protocol is designed for maximum yield potential covering nutritional aspects, biostimulation aspects and protection against diseases such as nematoes and fungi.

The process can be carried out by contacting soil to form a treated soil. In some cases the process is repeated. In some cases, plants, seedlings or seeds are already present in the soil prior to treatment with the microbial composition. In other cases, plants, seedlings or seeds are transplanted to the soil after treatment with the microbial composition.

In general, before application the number of hectares or acres to be treated is determined. Then the recommended amount of activated HYTa per hectare or acre is multiplied by the area to be treated and diluted in sufficient water to irrigate or spray the soil or crop on the area to be treated. The same procedure can be followed for liquid HYTb. HYTc, being a solid, can be applied directly as a solid or as a suspension in water. HYTc is preferably ground to micron size particles prior to use.

The process can be carried out with infertile soil. Such soils generally are those were at least one of low cation exchange capacity, low water holding capacity, low organic matter content and low levels of available nutrients is present. In general, infertile soil does not support vigorous plant growth and/or produces low crop yields.

For non-soil systems such as hydroponics, the same protocol applies but with a daily distribution following the ferti-irrigation program.

The microbial compositions can be used in connection with any plant including but not limited to alfalfa, banana, barley, broccoli, carrots, corn, cucumber, garlic, grapes, leek, melon, onion, potato, raspberry, rice, soybean, squash, strawberry, sugarcane, tomato and watermelon.

When applied as a soil amendment, the microbial composition containing HYTa, chitin, amino acids and chitosan enhances crop production on average about 25%-55% as compared to the 15-25% increase in crop production observed for E2001. From Karl Co. SA de CV, Navojoa, Sonora, Mexico.

The microbial composites can also result in a decrease in the amount of chitin used. For example, chitin has been used as a soil amendment in the prior art. Typically, about 600 kg of chitin were used per hectare. However, beneficial effects of such use were not observed for up to six months. When HYTa was activated in the presence of chitin and then combined with chitin and applied as a soil amendment, beneficial effects were observed after seven days with the use of only 4-6 kg of chitin per hectare.

Although the disclosure is directed primarily to the use of the disclosed microbial compositions for agricultural applications, such compositions or their components and processes can also be used in horticultural applications to improve the production of foliage and flowers and decrease the use of conventional insecticides and fungicides.

When activated HYTa is applied to soil, seed seedling or foliage it forms treated soil, treated seed, treated seedling, treated foliage and treated plants. HYTa is a novel microbial composition. Therefore the soil, seed, seedling, foliage and plants treated with HYTa are also novel.

Treated soil is defined as soil that contains one or more microbes that are unique to HYTa dispersed within the treated soil. Such microbes can be detected in the treated soil genetically by using a BioChip that detects microbial populations based on DNA. See e.g. US Patent Publication 2007/0015175. Other methods, such as PCR, which know to those skilled in the art can also be used. Microbes in HYTa that are particularly preferred are *Bacillus subtilis* (SILoSil® BS), *Bacillus thuringiensis* strain HD-1, *Bacillus thuringiensis* strain HD-73 (SILoSil® BT) and *Trichoderma harzianum* (TRICHOSIL) each of which can be isolated from the HYTa deposit or obtained from Biotecnologia Agroindustrial S.A. DE C.V., Morelia, Michoacan, Mexico. *Trichoderma harzianum* (TRICHOSIL) is most preferred as it is important during the activation of HYTa in that it causes inter-component synergies among the other microbes in HYTa. Identification of one or more of these microorganisms can be further combined with the identification of other microbes in HYTa, if necessary, to confirm the presence of HYTa or that HYTa was present. Each of *Bacillus subtilis* (SILoSil® BS), *Bacillus thuringiensis* strains HD-1 and HD-73 (SILoSil® BT) and *Trichoderma harzianum* (TRICHOSIL) were deposited with the ATCC.

Treated seed, seedlings, foliage and plants are similarly defined. In these cases, the microbes of HYTa are found on the surfaces of the treated seed, seedlings, foliage and plants.

As used herein, the term "consisting essentially of " in connection with HYTa, HYTb and HYTc means any of HYTa, HYTb and/or HYTc alone or in combination without additional microbes.

### Example 1

The following example compares the growth of Persian cucumber plants using HYTa, HYTb and chitosan as compared to a control which was not treated with HYTa, HYTb and chitosan.

During the development of seedlings of Persian cucumber, seeds were incubated for three hours in a mixture of 1 liter of water and 250 grams of HYTc. A bag of peat moss and 250 grams of micronized 200 mesh (approximately 75 micron diameter) chitin (HYTc) per bag of peat moss were blended. The seeds were planted in the peat moss/chitin mixture at 18-24°C. The plant development after five days following treatment with HYTc was comparable to 9 days of development without the treatment.

The treated and control seedlings were transplanted into 1 hectare of soil in a green house. The HYTa and control soil were treated as set forth in Table 5

**Table 6**

| | **HYT-A** | **Contr ol** |
|---|---|---|
| Nitrogen fertilizer | 150 Kg | 280 Kg |
| Potash | 160 Kg | 250 Kg |
| Calcium | 80 Kg | 130 Kg |
| Phosphorous | 200 Kg | 320 Kg |
| Magnesium | 20 Kg | 45 Kg |
| Trace elements | 10 liters | 22 liters |
| Fungicides | 0 | 20 liters |
| Insecticides | 0 | 16 liters |
| agricultural soap made from palm and olive oil | 10 iters | 0 |

The soil containing the HYTa treated seedlings was treated with 2 liters HYTa and 7 liters of HYTb over time.

HYTa was diluted in 200 liters of water and activated without the presence of HYTb or HYTc.

At week two, one liter of HYTa and three liters of HYTb were applied to the soil and two liters of HYTb were applied to the foliage of the HYT treated plants.

There was a significant increase in the yield of cucumbers over the control. The control plants produced 3,000 twenty five pound boxes while the HYT treated plants produced 4,300 boxes. Accordingly, this example demonstrates a significant increase in yield using HYT and a decrease in the amount of fertilizer, insecticides, fungicides and other components otherwise needed.

### Example 2

Septoria leaf and early blight as well as infection of Roma and beefsteak tomatoes with *Phytophthorainfestans* can be treated by the protocol set forth in Table 7. All values are per hectare.

**Table 7**

| | **Start** | **Per day** | **Duration** | **Application** |
|---|---|---|---|---|
| HYTa | 3 litres | 0 | 10 days | Spraying |
| HYTa | 2 litres | 0 | 10 days | Drip System |
| HYTc | 20 Kg | 2 Kg | 10 days | Spraying |
| HYTc | 500 grms | 0 | 0 | Drip System |
| HYTb | 1 liter | 1 liter | 10 days | Spraying |

HYTa was diluted in 200 liters of water and activated with HYTc.

This treatment resulted in control of these infections.

### Example 3

10 acres of Roma tomatoes were treated with 4 liters of HYTa, 10 liters of HYTb and 30 pounds of chitin.

The application protocol was as follows for 10 acres:

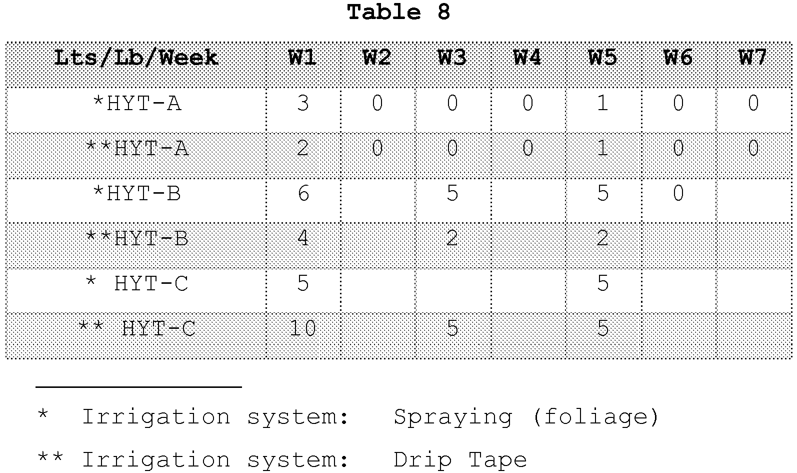

The values are in liters for HYTa and HYTb and pounds for HYTc. The crop yield was 46 tons of tomatoes per acre as compared to 31 tons per acre for the control. This is a 36% increase in yield.

### Example 4

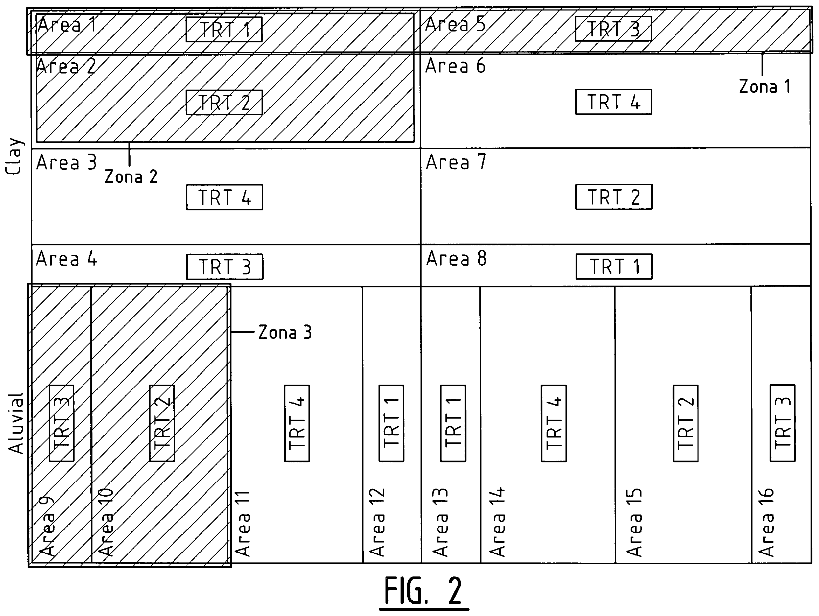

Root-Knot nematode *Meloidogyne* spp. and white mold disease caused by *Sclerotiniasclerotiorum* were identified as problematic for the growth of carrots. Figure 2A shows the foliage and carrots obtained from such soil.

The following protocol was used to treat a hectare. One Kg of HYTc was applied to the soil at the time of transplantation. Two weeks later 1 Kg of HYTc and 1.5 liters of HYTa was applied. Two weeks later 2 Kg of HYTc and 1 liter of HYTb was applied. Thirty days later 1.5 Kg of HYTc, 1 liter of HYTb and 1 liter of HYTa were applied.

The root galls caused by the nematode infection was no longer present on the carrots after the treatment. The cottony soft rot caused by white mold was also absent from the carrots after treatment.

### Example 5

HYTa, HYTb and HYTc can be used to eradicate and control ROYA (*Puccinia dracunculina*) on Tarragon (*Artemisia dracunculus L*.). A total of 6 liters of HYTa, 15 liters of HYTb and 900 grams of HYTc were applied to each hectare.

The following protocol was used:

**Table 9**

| Product | Dosage | Round time | Application |
|---|---|---|---|
| **HYTa** | 2 liters | 5 days | spraying |
| **HYTb** | 5 liters | 5 days | spraying |
| **HYTc** | 300 grms | 5 days | spraying |

The protocol was repeated twice. This treatment reduced damage from ROYA on treated foliage.

### Example 6

This example discloses a summary of tests carried in cooperation with and under the supervision of the Centre International of Maize and Wheat Improvement Center (known and referred to herein as the "CIMMYT") http:///www.cimmyt.org/.

This example presents the final data from the harvest of the different treatments. CIMMYT staff performed the collection of samples in accordance with its scientific methodologies and information.

These tests were designed to demonstrate the following key benefits of using HYTa alone or in combination with HYTb: (1) the ability to maintain high-performance growth with different regimes of fertilizer and minerals, (2) improving the performance of the system through the use of HYTa or HYTa in combination with HYTb, and (3) the ability to restore soil health soil and increased the levels of fertility through the repeated use of HYT programs.

The objective of the test was to determine the effect of the levels of tillage and the handling of straw in two different environments of soils (neighborhood and alluvium), investigate the efficiency of the different forms, types and doses of mineral fertilizers in combination with HYT of Agrinos to make more efficient use of these inputs in order to increase the profitability of the cultivation of wheat to the producer.

### Areas of test

These trials were performed in an agricultural field associated with the assignee which has been used widely for the development of soil remediation products, as well as for the production of cash crops. This agricultural field was treated over the last eleven years with E2001 and related products from Karl Co. SA de CV, Navojoa, Sonora, Mexico and more recently with HYTa and HYTb

This area of trials is identified by CIMMYT under the coupon code Z 702 Module Agirnos-CIMMYT and is in the District of irrigation Nr. 38, module 4, section 15, rolls of irrigation 1049-0 and 1115-0.

One of the main attributes of HYT ™ products is its ability to improve (instead of degrade) agricultural soils with continuous use. In order to demonstrate this attribute of the HYT™ product, the trials have included a test area which was not treated with mineral inputs, E 2001 or any HYT product. The performance of plants of the crop in this area depends entirely on the state of the soil prior to planting.

### Other information

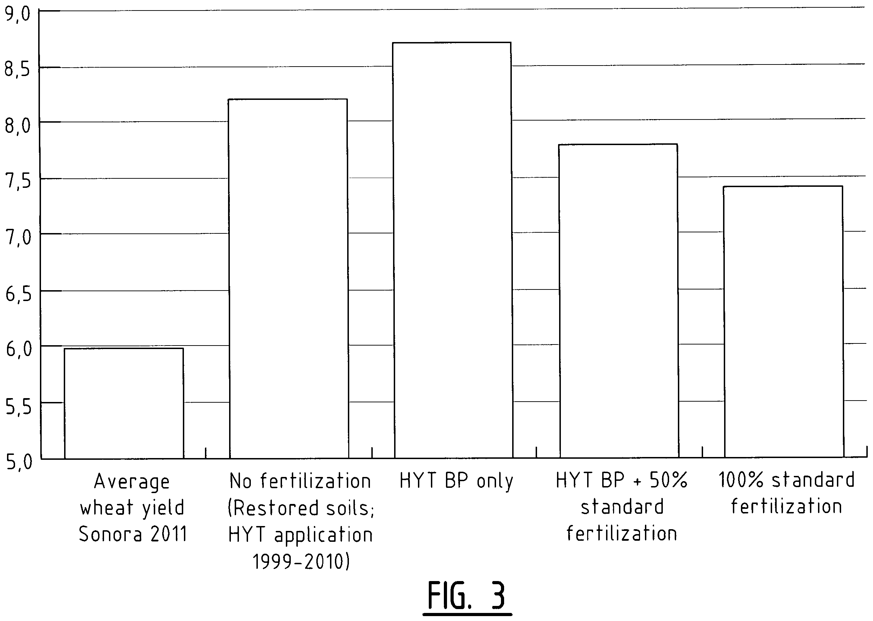

- Types of crops: wheat
- Variety: ATIL (durum wheat)
- Sowing date: 23 December in dry soil and wet soil in areas 2 and 1 of Figure 3. Planting was delayed until January 14, of the following year due to a flood caused by an irrigation problem in the adjoining plot.
- Date of harvest: May 20-23, (approximately 4 months after planting)
- Size of the test area: 15 hectares
- Mineral fertilization: was used as the basis for fertilization which is considered as the best practice of deossification of mineral nutrients NPK generally accepted in the region (BNFP = Best nitrogen fertilizer practice).

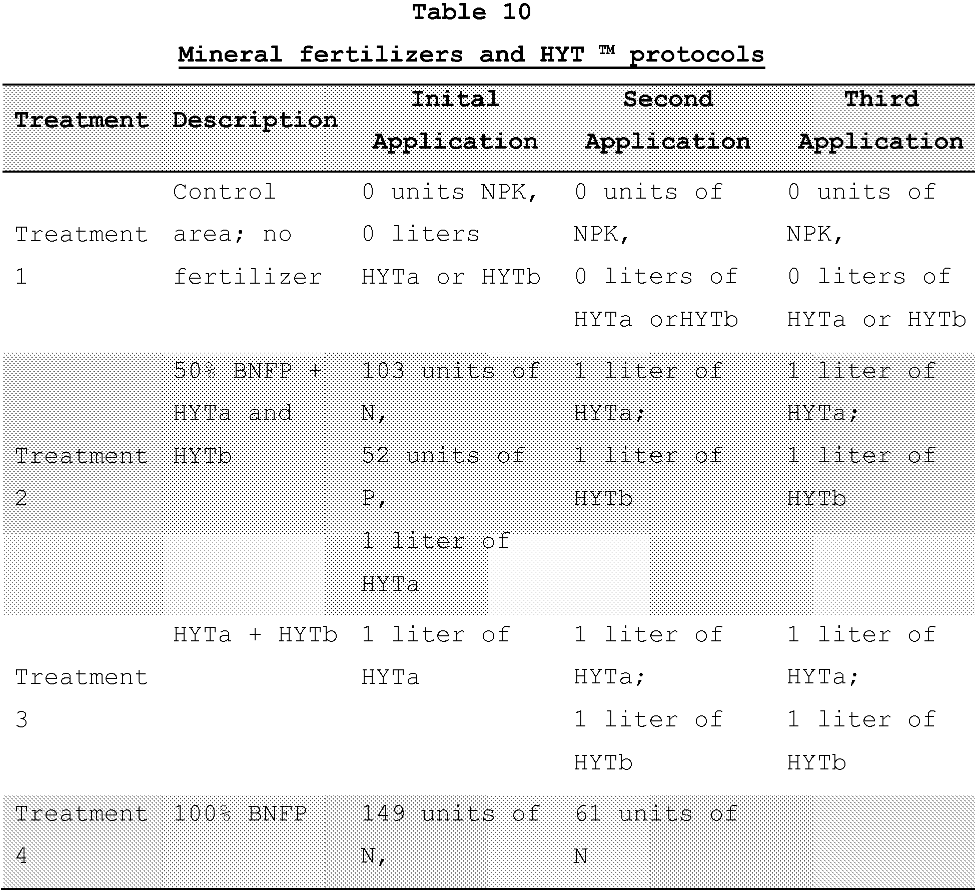

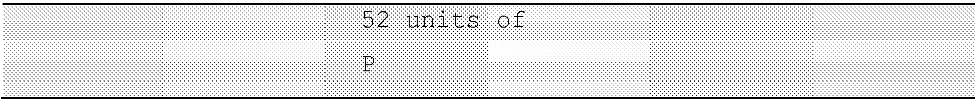

In addition to the main protocols described above, some areas were tested and harvested separately with some additional component, with the aim of obtaining an extra point of reference and expand the possibilities for analysis. The designated test was as follows:
TRT 5: Biological treatment HYT more 100% traditional mineral fertilization programme: initial application: 1 litre of HYT + 103-52-0 (NPK), second application 1 litre of HYT + 1 litre of HYT B + 61-0-0 (NPK), third implementation 1 liter of HYT B. This further treatment was recommended by CIMMYT in order to observe the behavior of the traditional program of more complete mineral nitrogen program HYT a + b in the performance of the grain of wheat. Only an area of 4 rows was dedicated to this treatment and the information was collected only by CIMMYT staff.

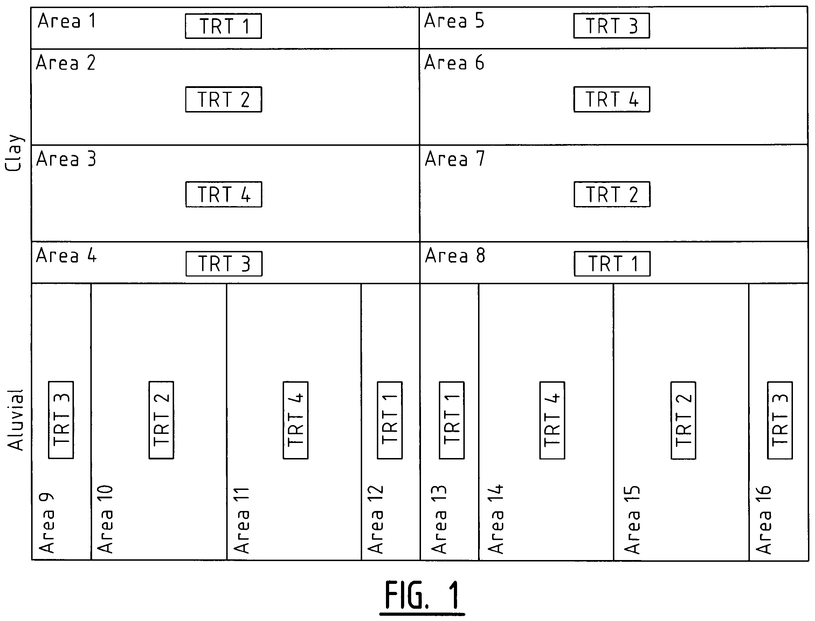

A diagram of the test area is shown in Figure 1 where "TRT" refers to the above identified treatment.

### External factors in the test area

Some areas of the test zone were compromised and suffered impairment by external factors. The results of these areas have been excluded from the final results of the harvest to allow a reliable comparison. Reference is made to Figure 2. These external factors were as follows:
Area highlighted 1 (Zone 1): The wheat variety used is very susceptible to "Chahuistle". Due to the proximity of areas 1 and 5 to high voltage electric lines, the plane could not apply the product on these areas and consequently these areas suffered a higher incidence of the pathogen, causing a significant loss of performance potential.

Highlighted areas 1 and 2 (Zone 2) suffered flooding due to problems of irrigation in the surrounding plots, delaying the planting in 20 days and being affected by the "chahuistle".

Highlighted areas 9 and 10 (Zone 3) suffered from irregular irrigation due to the topography of the ground which makes erratic performance of the crop having low and high areas causing non-uniform irrigation that affects the average of the performance.

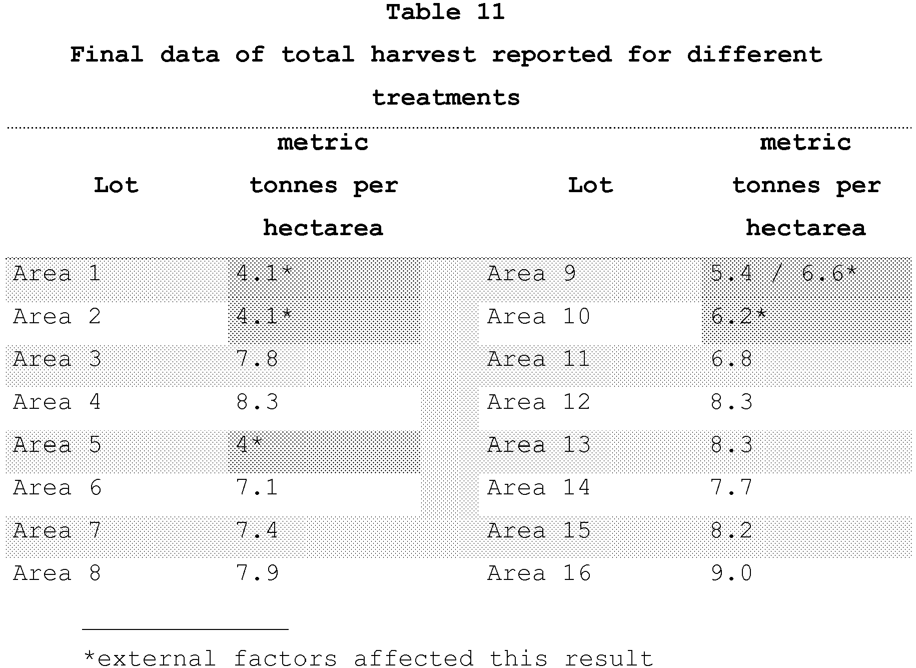

**Table 12**

| **Results of Trial** | | | |
|---|---|---|---|
| Performance | | | |
| Tonnes/Ha* | **Clay*** | **Alluvial*** | **Average*** |
| 100% BNFP (Treatment 4) | 7.45 | 7.40 | **7.4** |
| 50% BNFP plus HYTa and HYTb (Treatment 2) | 7.40 | 7.20 | **7.8** |
| Control (Treatment 1) | 7.90 | 8.30 | **8.2** |
| HYTa and HYTb only (Treatment 3) | 8.30 | 6.50 | **8.7** |

| | | | |
|---|---|---|---|
| *results from areas where external factors affected results are not included | | | |

In comparison with the average wheat yield expected in the region the repeated historical use E 2001 and HYT ™ has contributed to the significant increase in performance of 36% as compared to standard fertilization only. See Figure 3. This happened without adding any additional element of NPK or HYT during this agricultural cycle given that previous E 2001 and HYT applications had already restored the activity and biodiversity of colonies of benign soil microbes creating high levels of organic matter and nutrients available in the soil.

Adding the HYTa and HYTb to the soil-plant cycle system continues the improvement of the capacity of the soil to provide nutrients to plants, increasing the capacity of biological nitrogen fixation. See Figure 3.

Various combinations of standard fertilization regimes, alone or in combination with HYTa and HYTb do not seem to improve the results as compared to the use of HYTa and HYTb. This may be due to the existence of sufficient stored nutrients as biomass in the soil from previous years.

When the soil and ecosystem have sufficient available nutrients, either through FBN and/or high levels of biomass in the soil, the addition of more (NPK) fertilizer destabilized the biological balance and interfered with the patterns of absorption of nutrients from the plants, possibly changing the capacity of the cultivation of guiding their own nutritional program given the resources available in the soil and active biomass in its roots.

### Example 7

Field experiments were conducted at Pantnagar India under the project entitled 'Agronomic evaluation of HYT (HYTa, HYTb and HYTc and foliar spray of Suryamin). The details are given below.
**Crop** : WHEAT
**Design used** : RBD
**Replication** : 3
**Date of sowing** : 19.11.2010
**Variety** : PBW-550
**Gross plot size** : 6.0 m x 4.0 m= 24 m²
**Treatments** : 12

### Treatment details

**T-₁:** Recommended NPK dose
**T-₂:** T-1+soil application of HYTa (activated for 72 hrs) @ 1L at the time of sowing
**T-₃:** T-1+ foliar application of HYTb @ 2L at the time of flower initiation/ panicle initiation)
**T-₄:** T-1+soil application of HYTc @ 2 kg at the time of sowing
**T-₅:** T-1+ HYTa (activated for 72 hrs) @ 1Liter + HYTc@ 2kg/ha as soil application at sowing
**T-₆:** T-1+ foliar application of HYTb @ at the time of flower initiation/ panicle initiation + 2L+HYTc @ 2kg/ha as soil application at sowing
**T-₇:** T-1+HYTa (activated for 72 hrs) @1L +HYTb @ 2L at the time of flower initiation/ panicle initiation+ HYTc @2 kg/ha as soil application at sowing
**T-₈:**½ NPK dose + HYTa (activated for 72 hrs) @ 1L+ foliar application of HYTb @ 2L+HYTc @ 2kg/ha as soil application at sowing
**T-₉:** T-1+ soil application of HYTa (activated for 72 hrs) @ 2L/ha+ foliar application of HYTb @ 5L+HYTc @ 5kg/ha at sowing
**T-₁₀:** ½ NPK dose +HYTa (activated for 72 hrs) @ 2L+foliar application of HYTb @ 5L+HYT-C @ 5kg/ha as soil application at sowing
**T-₁₁:** T-1+ 1L/ha of Shriram Suryamin as foliar application at flower initiation
**T-₁₂:** T-1+1L/ha of Shriram Suryamin as foliar application each at tillering and at flower initiation The biological, grain and straw yields are set forth in Table 13.

**Table 13**

| **Effect of different HYT organic product on biological, grain and straw yield of wheat crop.** | | | |
|---|---|---|---|
| **Treatments** | **Biological Yield (q/ha)** | **Grain Yield (q/ha)** | **Straw Yield (q/ha)** |
| T₁: Rec. NPK | 82.63 | 32.00 | 50.63 |
| T₂: T₁ + HYT-A @ 1.0 l/ha | 85.43 | 33.99 | 51.50 |

| **Treaments** | **Biological Yield (q/ha)** | **Grain Yield (q/ha)** | **Starw Yield (q/ha)** |
|---|---|---|---|
| T₃: T₁ + HYT-B@ 2.0 l/ha | 87.50 | 35.30 | 52.20 |
| T₄: T₁ + HYT-C @ 2.0 kg/ha | 76.40 | 31.33 | 45.07 |
| T₅: T₁ + HYT-A +C | 87.63 | 37.90 | 49.73 |
| T₆: T₁ + HYT-B+C | 84.53 | 34.80 | 49.73 |
| T₇: T₁ + HYT-A +B+C | 86.93 | 35.80 | 51.93 |
| T₈: ½ NPK+HYT-A+B+C | 56.90 | 27.13 | 29.77 |
| T₉: T₁ + HYT-A +B+C (higher dose) | 88.60 | 40.27 | 48.33 |
| T₁₀: ½ NPK+HYT-A+B+C (higher dose) | 58.37 | 28.30 | 30.07 |
| T₁₁:T₁+ Suryamin (one spray) | 82.87 | 32.93 | 49.93 |
| T₁₂ :T₁+ Suryamin (two spray) | 83.57 | 33.17 | 50.40 |
| S.Em (5%) | 4.33 | 0.92 | 4.27 |
| CD (5%) | 12.68 | 2.71 | 12.54 |

Table 14 compares the results for grain yield for the various treatments with the different HYT components and combinations.

**Table 14**

| | **Grain Yield above 32 Kilo/hectare** | **CD 2.71** | **Std. Error 0.92** |
|---|---|---|---|
| Control | 0 | | |
| HYTa | 1.99 | NS | * |
| HYTb | 3.3 | * | * |
| HYTc | -0.67 | NS | NS |
| HYTa + c | 5.9 | * | * |
| HYTb + c | 2.8 | * | * |
| HYTa + b + c (1L/2L/2Kg) | 3.8 | * | * |
| HYTa + b +c (high dose of a, b and c: 2L/5L/5Kg) | 8.27 | * | * |

| | | | |
|---|---|---|---|
| NS = Not statistically significant as compared to control * = Statistically significant as compared to control | | | |

As can be seen, the separate use of HYTa and HYTb improved grain yield by 1.99 and 3.3 kilo per hectare respectively while the use of HYTc alone caused a decrease in yield. When HYTa was combined with HYTc the yield increase was 5.9 kilo per hectare which is greater than the sum of the results when used separatly. The use of HYTb and HYTc resulted in an increase of 2.8 kilo per hectare ahile the use of HYTa, HYTb and HYTc caused an increase of 3.8 kilo per hectare. The greatest increase in grain yield was observed for HYTa, HYTb and HYTc used at the higher doses indicated. This resulted in an increase of over 25% in grain yield over the control, i.e. an 8.3 kilo per hectare increase.

### Example 8

This example sets forth results of the growth of squash in infertile soil.

In these experiments squash seedlings were planted in 5 gallon pots containing infertile "Superstition" sand. The combinations of HYT A, B, and C were applied as set forth in Table 16. The seedlings were planted December 21 and harvesting began January 20 and continued through February 27 of the following year.

**Table 15**

| **Date** | **Treatment rates applied** |
|---|---|
| December 16 | 200 mL activated HYT A, 10 mL HYT B, and 200 g HYT C per pot. |
| December 23 | 10 mL HYT B per pot. |
| December 30 | 5 mL HYT B per pot. |
| January 5 | 5 mL HYT A and 5 mL HYT B per pot. |
| January 19 | 10 mL HYT B per pot. |
| January 27 | 10 mL HYT A and HYT B per pot. |
| February 3 | 10 mL HYT B per pot. |
| February 10 | 10 mL HYT A and 10 mL HYT B |
| Feb. 17 | Applied 5 mL HYT A and 5 mL HYT B to each pot. |
| Feb. 25 | Applied 5 mL HYT B to each pot. |
| March 1 | Applied 5 mL HYT A and 5 mL HYT B to each pot. |

The results are set forth in Table 16.

**Table 16**

| **Treatment** | **Yield (g/pot)** |
|---|---|
| Control | 0 |
| HYTa | 251.6 |
| HYTb | 137.9 |
| HYTc | 62.6 |
| HYTa + HYTb | 472.1 |
| HYTa + HYTc | 0 |
| HYTb + HYTc | 0 |
| HYTa + HYTb + | 62.3 |
| HYTc | |
| Stat. A | ** |
| B | ** |
| C | NS |
| A*B | NS |
| A*C | ** |
| B*C | NS |

| | |
|---|---|
| NS is not significant at P<0.05. *, ** are statistically significant at P<0.05 and P<0.01, respectively. | |

As can be seen, there was a substantial increase in yield of zucchini squash when HYTa and HYTb were separately used and when HYTa and HYTb were used in combination.

### Example 9

The following protocol was used to treat melons.

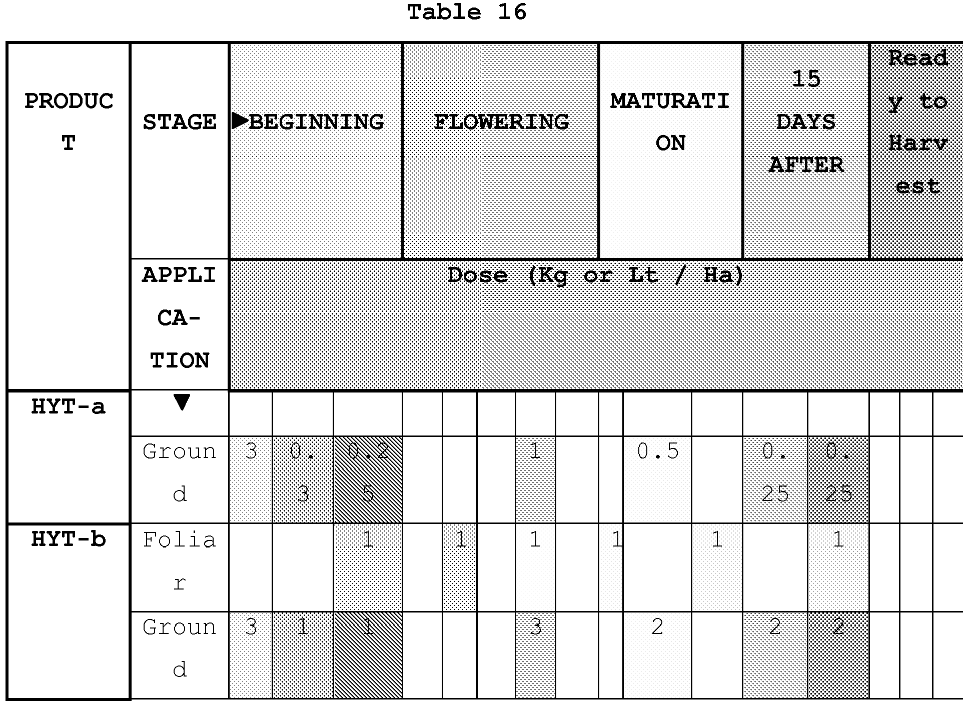

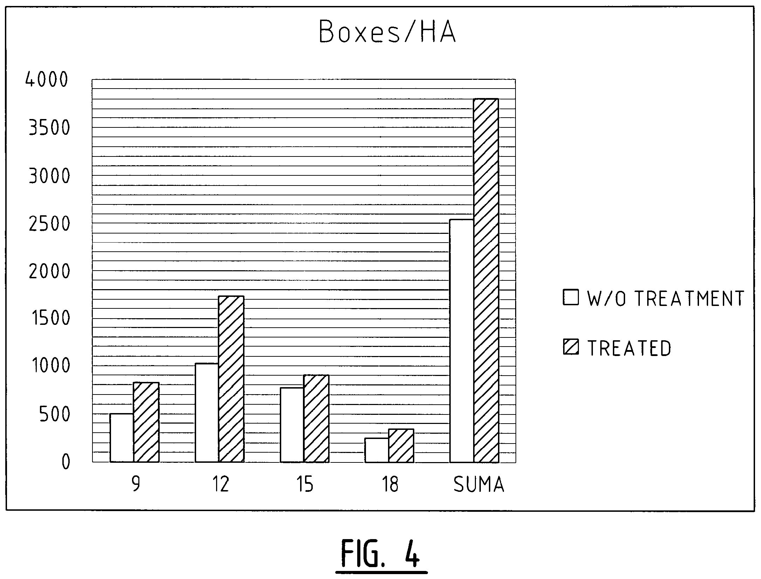

The results are set forth in Table 17 and Figure 4.

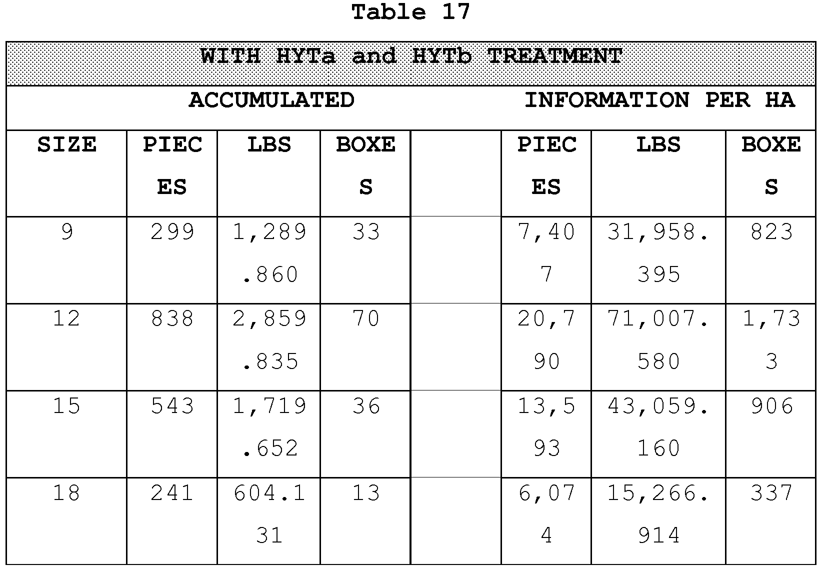

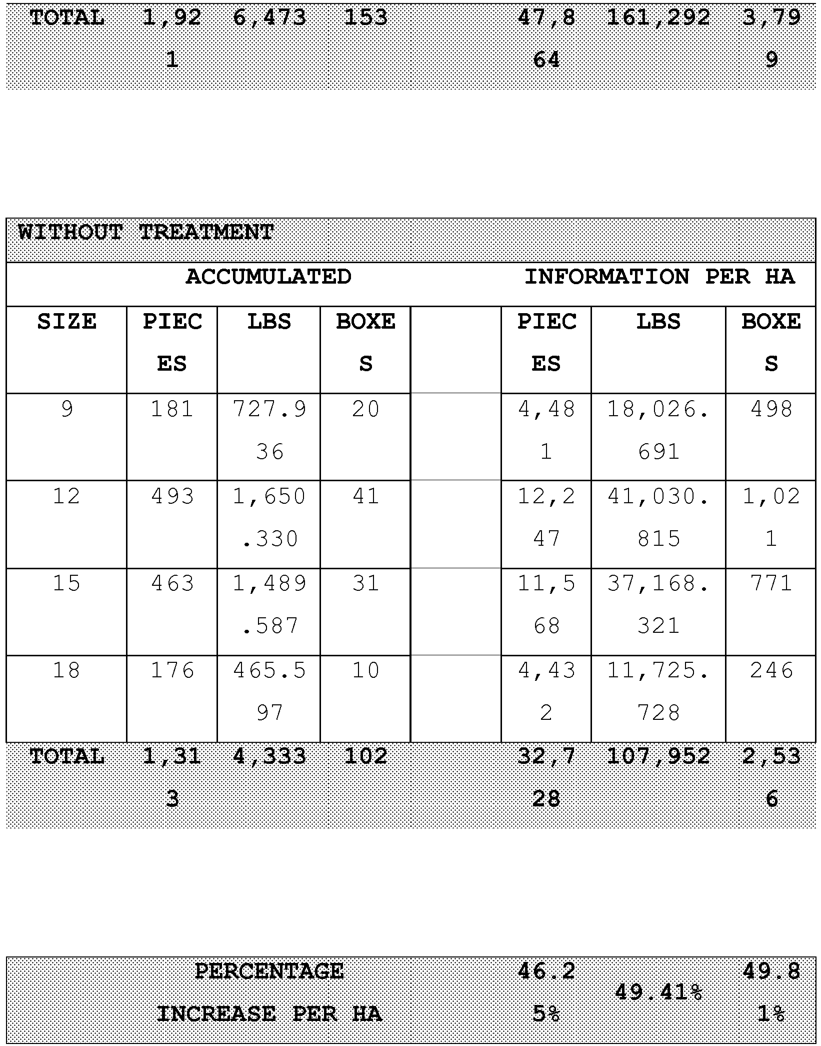

### Example 10

Squash were grown according to the following protocol.

**Table 18**

| **PRODUCT** | **APPLICATION** | **DOSE PER HECTARE** | | | | | |
|---|---|---|---|---|---|---|---|
| | | **03/07** | **03/10** | **03/18** | **04/04** | **04/11** | **04/18** |
| **Hyt-a** | Soil | 2 | | | 1 | | |
| **Hyt-b** | Foliar | | 1 | 1 | 3 | 1 | 1 |
| | Soil | 1 | 1 | 1 | | 1 | 1 |

The results are set forth in Table 19

**Table 19**

| | **BOXES/HA** | | **DIFFERENCE IN BOXES/HA** | **DIFFERENCE IN %** |
|---|---|---|---|---|
| **SIZE** | **CONTROL** | **TREATED.** | | |
| X | 188 | 228 | 40 | 17.54% |
| XX | 63 | 145 | 82 | 56.55% |
| XXX | 47 | 95 | 48 | 50.52% |
| BRUCE | 29 | 30 | 1 | 3.33% |

### Example 11

A trial with HYTa and HYTb was conducted in Norway on a potato crop. Tests with and without HYTa and HYTb were treated with 50 or 100 kg Nitrogen fertilizer/ha. Pesticides were used in normal amounts.

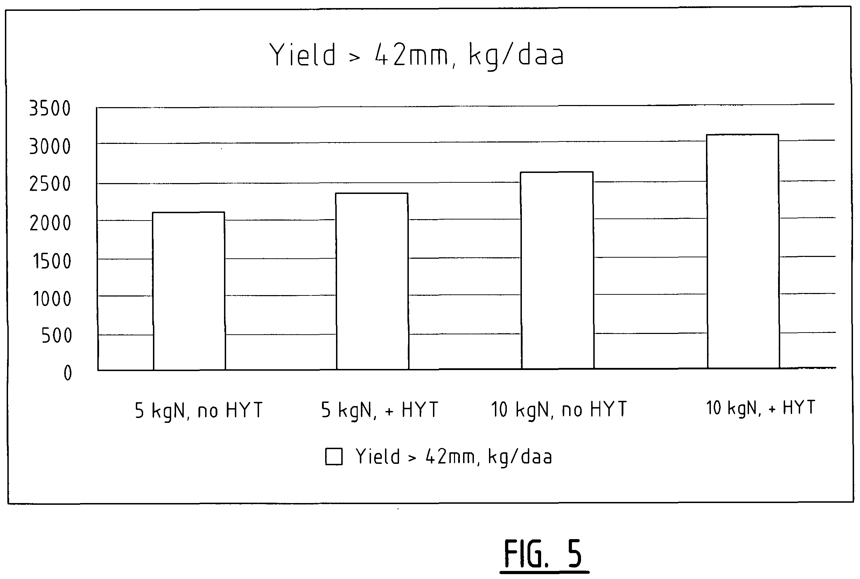

At the time of first emergence (June 14), 0.2 liers of HYTa and 0.6 liters of HYTb was applied per decare. After the last application of dirt (July 20), 0.2 liter of HYTa, 0.2 liter of HYTb and 50 grams of HYTc were applied per decare. The results are shown in Figure 5

Use of HYTa and HYTb gave a yield increase of up to 17% as compared to the control. In addition, there was less potato blight on the HYTa and HYTb treated crop as compared to control.

## Claims

1. A microbial composition comprising (i) HYTa and (ii) at least one of chitin, chitosan, glucosamine, and amino acids, wherein said HYTa comprises the microbes in ATCC Patent Deposit designation PTA-10973.

2. The microbial composition of claim 1 comprising HYTa and at least two, three, or all of chitin, chitosan, glucosamine, and amino acids.

3. The microbial composition of claim 1 or 2, wherein said chitin is derived as a solid from the biodegradation of chitin containing *Arthropods,* and said chitosan, glucosamine, and amino acids are derived from the aqueous fraction from the biodegradation of chitin-containing *Arthropods.*

4. The microbial composition of any one of claims 1 to 3, wherein said composition comprises chitin, and wherein said chitin is from HYTc, wherein said HYTc comprises the solid fraction obtained from the biodegradation of chitin containing *Arthropods* with a microbial composition comprising the microbes in HQE, and wherein said HQE comprises the microbes in ATCC Patent Deposit designation PTA-10861.

5. The microbial composition of any one of claims 1 to 4 wherein said composition comprises one or more of chitosan, glucosamine, and amino acids, and wherein the chitosan, glucosamine, and amino acids are from HYTb, wherein said HYTb comprises the aqueous fraction obtained from the biodegradation of chitin containing *Arthropods* with a microbial composition comprising the microbes in HQE, and wherein said HQE comprises the microbes in ATCC Patent Deposit designation PTA-10861.

6. A microbial composition comprising HYTa and at least one of HYTb and HYTc, wherein said HYTb comprises the aqueous fraction obtained from the biodegradation of chitin containing *Arthropods* with a microbial composition comprising the microbes in HQE, wherein said HQE comprises the microbes in ATCC Patent Deposit designation PTA-10861 and said HYTc comprises the solid fraction obtained from the biodegradation of chitin containing *Arthropods* with a microbial composition comprising the microbes in HQE.

7. The microbial composition of claim 6, wherein the composition comprises HYTa, HYTb and HYTc.

8. The microbial composition of claim 6 or 7, wherein the composition consists of HYTa, HYTb, and HYTc.

9. A process comprising contacting soil, seed, seedling, or plant foliage with (i) HYTa, wherein said HYTa comprises the microbes in ATCC Patent Deposit designation PTA-10973, or (ii) a microbial composition as defined in any one of claims 1-8.

10. The process of claim 9, wherein the HYTa is activated in an aqueous solution for 24-168 hours before said contacting.

11. Activated HYTa made by incubating a microbial composition comprising HYTa for 24-168 hours, preferably made by incubating a microbial composition comprising HYTa in the presence of HYTc for 24-168 hours, where said HYTa comprises a microbial composition comprising the microbes in ATCC Patent Deposit designation PTA-10973 and wherein said HYTc comprises the solid fraction obtained from the biodegradation of chitin containing *Arthropods* with a microbial composition comprising HQE, wherein said HQE comprises the microbes in ATCC Patent Deposit designation PTA-10861.

12. A process comprising applying a mixture of the activated HYTa of claim 11 and at least one of HYTb and HYTc to soil, foliage, seed, or seedlings.

13. The process of claim 12, comprising applying the activated HYTa of claim 11, HYTb, and HYTc to soil, foliage, seed, or seedlings.

14. A composition comprising soil, a plant, seed or seedling and a microbial composition comprising the microbes in HYTa, wherein the microbes in HYTa comprise the microbes in ATCC Patent Deposit designation PTA-10973.

## Patentansprüche

1. Mikrobielle Zusammensetzung, die (i) HYTa und (ii) zumindest eines aus Chitin, Chitosan, Glucosamin und Aminosäuren umfasst, wobei HYTa die Mikroben in der ATCC-Patenthinterlegungsnr. PTA-10973 umfasst.

2. Mikrobielle Zusammensetzung nach Anspruch 1, umfassend HYTa und zumindest zwei, drei oder alle von Chitin, Chitosan, Glucosamin und Aminosäuren.

3. Mikrobielle Zusammensetzung nach Anspruch 1 oder 2, wobei das Chitin als Feststoff aus dem biologischen Abbau von Chitin enthaltenden Arthropoda abgeleitet ist und das Chitosan, Glucosamin und die Aminosäuren aus dem wässrigen Teil des biologischen Abbaus von Chitin enthaltenden Arthropoda abgeleitet ist.

4. Mikrobielle Zusammensetzung nach einem der Ansprüche 1 bis 3, wobei die Zusammensetzung Chitin umfasst und wobei das Chitin aus HYTc stammt, wobei HYTc den festen Teil aus dem biologischen Abbau von Chitin enthaltenden Arthropoda mit einer mikrobiellen Zusammensetzung umfassend die Mikroben in HQE umfasst und wobei das HQE die Mikroben in der ATCC-Patenthinterlegungsnr. PTA-10861 umfasst.

5. Mikrobielle Zusammensetzung nach einem der Ansprüche 1 bis 4, wobei die Zusammensetzung eines oder mehr aus Chitosan, Glucosamin und Aminosäuren umfasst und wobei das Chitosan, Glucosamin und Aminosäuren aus HYTb stammen, wobei HYTb den wässrigen Teil aus dem biologischen Abbau von Chitin enthaltenden Arthropoda mit einer mikrobiellen Zusammensetzung umfassend die Mikroben in HQE umfasst und wobei das HQE die Mikroben in der ATCC-Patenthinterlegungsnr. PTA-10861 umfasst.

6. Mikrobielle Zusammensetzung, umfassend HYTa und zumindest eines aus HYTb und HYTc, wobei HYTb den wässrigen Teil aus dem biologischen Abbau von Chitin enthaltenden Arthropoda mit einer mikrobiellen Zusammensetzung umfassend die Mikroben in HQE umfasst, wobei HQE die Mikroben in der ATCC-Patenthinterlegungsnr. PTA-10861 umfasst und HYTc den festen Teil aus dem biologischen Abbau von Chitin enthaltenden Arthropoda mit einer mikrobiellen Zusammensetzung umfassend die Mikroben in HQE umfasst.

7. Mikrobielle Zusammensetzung nach Anspruch 6, wobei die Zusammensetzung HYTa, HYTb und HYTc umfasst.

8. Mikrobielle Zusammensetzung nach Anspruch 6 oder 7, wobei die Zusammensetzung aus HYTa, HYTb und HYTc besteht.

9. Verfahren, das das Kontaktieren von Boden, Samen, Keimlingen oder Pflanzenblättern mit (i) HYTa, wobei HYTa die Mikroben in der ATCC-Patenthinterlegungsnr. PTA-10973 umfasst, oder (ii) einer mikrobiellen Zusammensetzung, wie sie in einem der Ansprüche 1 bis 8 definiert ist, umfasst.

10. Verfahren nach Anspruch 9, wobei HYTa vor dem Kontaktieren in wässriger Lösung 24 bis 168 h lang aktiviert wird.

11. Aktiviertes HYTa, hergestellt durch das 24 bis 168 h lange Inkubieren einer mikrobiellen Zusammensetzung, die HYTa umfasst, vorzugsweise hergestellt durch das 24 bis 168 h lange Inkubieren einer mikrobiellen Zusammensetzung, die HYTa umfasst, in Gegenwart von HYTc, wobei das HYTa eine mikrobielle Zusammensetzung umfassend die Mikroben in der ATCC-Patenthinterlegungsnr. PTA-10973 umfasst und wobei das HYTc den festen Teil aus dem biologischen Abbau von Chitin enthaltenden Arthropoda mit einer mikrobiellen Zusammensetzung umfassend HQE umfasst, wobei HQE die Mikroben in der ATCC-Patenthinterlegungsnr. PTA-10861 umfasst.

12. Verfahren, umfassend das Aufbringen eines Gemischs aus aktiviertem HYTa nach Anspruch 11 und zumindest einem aus HYTb und HYTc auf Boden, Blätter, Samen oder Keimlinge.

13. Verfahren nach Anspruch 12, umfassend das Aufbringen von aktiviertem HYTa nach Anspruch 11, HYTb und HYTc auf Boden, Blätter, Samen oder Keimlinge.

14. Zusammensetzung, umfassend Boden, eine Pflanze, Samen oder Keimlinge sowie eine mikrobielle Zusammensetzung, umfassend die Mikroben in HYTa, wobei die Mikroben in HYTa die Mikroben in der ATCC-Patenthinterlegungsnr. PTA-10973 umfassen.

## Revendications

1. Composition microbienne comprenant (i) du HYTa et (ii) au moins un élément parmi la chitine, le chitosane, la glucosamine, et des acides aminés, où ledit HYTa comprend les microbes sous la désignation de dépôt de brevet ATCC PTA-10973.

2. Composition microbienne selon la revendication 1 comprenant du HYTa et au moins deux, trois, ou tous les éléments parmi la chitine, le chitosane, la glucosamine, et des acides aminés.

3. Composition microbienne selon la revendication 1 ou 2, dans laquelle ladite chitine est dérivée sous forme de solide issu de la biodégradation d'arthropodes contenant de la chitine, et lesdits chitosane, glucosamine, et acides aminés sont dérivés de la fraction aqueuse issue de la biodégradation d'arthropodes contenant de la chitine.

4. Composition microbienne selon l'une quelconque des revendications 1 à 3, où ladite composition comprend de la chitine, et dans laquelle ladite chitine est issue de HYTc, dans laquelle ledit HYTc comprend la fraction solide obtenue à partir de la biodégradation d'arthropodes contenant de la chitine avec une composition microbienne comprenant les microbes dans du HQE, et dans laquelle ledit HQE comprend les microbes sous la désignation de dépôt de brevet ATCC PTA-10861.

5. Composition microbienne selon l'une quelconque des revendications 1 à 4, où ladite composition comprend un ou plusieurs parmi le chitosane, la glucosamine, et des acides aminés, et dans laquelle le chitosane, la glucosamine, et les acides aminés sont issus de HYTb, où ledit HYTb comprend la fraction aqueuse obtenue à partir de la biodégradation d'arthropodes contenant de la chitine avec une composition microbienne comprenant les microbes dans du HQE, et dans laquelle ledit HQE comprend les microbes sous la désignation de dépôt de brevet ATCC PTA-10861.

6. Composition microbienne comprenant du HYTa et au moins un parmi le HYTb et le HYTc, où ledit HYTb comprend la fraction aqueuse obtenue à partir de la biodégradation d'arthropodes contenant de la chitine avec une composition microbienne comprenant les microbes dans du HQE, où ledit HQE comprend les microbes sous la désignation de dépôt de brevet ATCC PTA-10861 et ledit HYTc comprend la fraction solide obtenue à partir de la biodégradation d'arthropodes contenant de la chitine avec une composition microbienne comprenant les microbes dans du HQE.

7. Composition microbienne selon la revendication 6, où la composition comprend du HYTa, HYTb et HYTc.

8. Composition microbienne selon la revendication 6 ou 7, où la composition consiste en du HYTa, HYTb, et HYTc.

9. Procédé comprenant la mise en contact de terre, graine, jeune plant, ou feuillage de plante avec (i) du HYTa, où ledit HYTa comprend les microbes sous la désignation de dépôt de brevet ATCC PTA-10973, ou (ii) une composition microbienne telle que définie dans l'une quelconque des revendications 1 à 8.

10. Procédé selon la revendication 9, dans lequel le HYTa est activé dans une solution aqueuse pendant 24-168 heures avant ladite mise en contact.

11. HYTa activé fabriqué en incubant une composition microbienne comprenant du HYTa pendant 24-168 heures, de préférence fabriqué en incubant une composition microbienne comprenant du HYTa en présence de HYTc pendant 24-168 heures, où ledit HYTa comprend une composition microbienne comprenant les microbes sous la désignation de dépôt de brevet ATCC PTA-10973 et où ledit HYTc comprend la fraction solide obtenue à partir de la biodégradation d'arthropodes contenant de la chitine avec une composition microbienne comprenant du HQE, où ledit HQE comprend les microbes sous la désignation de dépôt de brevet ATCC PTA-10861.

12. Procédé comprenant l'application d'un mélange du HYTa activé selon la revendication 11 et d'au moins un élément parmi du HYTb et HYTc à de la terre, du feuillage, une graine, ou des jeunes plants.

13. Procédé selon la revendication 12, comprenant l'application du HYTa activé selon la revendication 11, de HYTb, et de HYTc à de la terre, du feuillage, une graine, ou des jeunes plants.

14. Composition comprenant de la terre, une plante, une graine ou un jeune plant et une composition microbienne comprenant les microbes dans du HYTa, dans laquelle les microbes dans du HYTa comprennent les microbes sous la désignation de dépôt de brevet ATCC PTA-10973.
